# FASCICULE DE BREVET EUROPEEN

(11) **EP 0 594 511 B1**
(45) Date de publication et mention de la délivrance du brevet: **11.12.1996**
(21) Numéro de dépôt: 93420333.2
(22) Date de dépôt: 05.08.1993
(51) Int. Cl.: A61F 2/24

(54) **Valve cardiaque artificielle**
Herzklappenprothese
Heart valve Prosthesis

(30) Priorité: 11.08.1992 FR 9210088
(43) Date de publication de la demande: 27.04.1994
(73) Titulaire: FABRIQUE D'IMPLANTS ET D'INSTRUMENTS CHIRURGICAUX SOCIETE A RESPONSABILITE LIMITEE, F-43240 Saint Just Malmont (FR)
(72) Inventeur: Cuilleron, Jean, FR-42100 Saint Etienne (FR); Baudet, Eugène, F-33700 Merignac (FR)
(74) Mandataire: Dupuis, François

(56) Documents cités:
- EP-A- 0 113 681
- EP-A- 0 318 351
- EP-A- 0 327 790
- EP-A- 0 436 420
- EP-A- 0 465 383
- WO-A-92/21305
- FR-A- 2 663 533
- US-A- 5 061 278

## Description

L'invention concerne plus particulièrement, une valve du type de celle décrite dans la demande de brevet FR-A-2663533 dont le demandeur est également titulaire.

Pour l'essentiel, ce brevet enseigne une valve cardiaque comprenant un anneau à l'intérieur duquel sont articulées des ailettes incurvées. L'anneau présente des agencements diamétralement opposés aptes à assurer le déport des moyens d'articulation des ailettes, pour qu'en position d'ouverture maximum d'environ au moins 85°, ces dernières ne débordent que d'une valeur réduite dans le plan de l'anneau en délimitant, en combinaison avec le profil des ailettes, trois aires équivalentes permettant un flux central axial et laminaire. Les moyens d'articulation desdites ailettes sont intégrés dans l'épaisseur de l'anneau, de sorte que les extrémités des ailettes, au niveau des agencements, sont écartées en délimitant un espace entièrement libre pour ne pas limiter et gêner le flux central.

Selon l'invention, on a voulu améliorer ces caractéristiques de base. Notamment, il est apparu qu'au moment de la fermeture des ailettes, ces dernières tapent très légèrement contre la périphérie interne de l'anneau. Or, compte-tenu des fréquences de battement et par conséquent, du cycle d'ouverture et de fermeture des ailettes, il peut se produire un phénomène d'usure au niveau de la zone de contact desdites ailettes, avec le corps de l'anneau.

En outre, au niveau de cette zone de contact, en position de fermeture des ailettes, c'est-à-dire en position de butée contre l'anneau, il se produit une force de réaction qui est transmise directement au niveau de l'axe d'articulation. Cette force de réaction a pour effet d'agir défavorablement sur l'articulation des ailettes.

L'invention s'est fixée pour but de remédier à ces inconvénients, de manière simple, sûre, efficace et rationnelle.

Un des problèmes que se propose de résoudre l'invention est de supprimer toute force de réaction susceptible d'agir sur l'articulation des ailettes, d'une part, et d'éviter tout phénomène d'usure au niveau de l'anneau, d'autre part. On veut donc éviter que les forces engendrées lors de la fermeture des ailettes dans la zone de contact avec l'anneau sollicitent leur partie d'articulation et entraînent une usure des dites ailettes au niveau de cette zone de contact. Il s'avère important d'obtenir un phénomène d'amortissement en ayant pour objectif de diminuer les bruits et l'usure, tout en tenant compte des problèmes du rendement de l'écoulement du fluide sans perturber le flux sanguin.

Pour résoudre un tel problème, l'anneau présente un agencement périphérique interne sous forme d'une empreinte en creux, correspondant au profil externe du bord de l'ailette et dont la section transversale est constituée par un profil courbe engendré par le déplacement du bord externe des ailettes en position de fermeture et en créant un effet de butée.

Un autre problème que se propose de résoudre l'invention, est d'assurer la butée en position d'ouverture maximum des ailettes, d'une manière simple et efficace, en ayant pour objectif de rationnaliser la fabrication.

Un tel problème est résolu en ce que les agencements présentent des moyens de blocage en position d'ouverture des ailettes, sous forme d'une pastille rapportée fixée en débordement de la face interne de l'anneau, entre les parties d'articulation desdites ailettes.

Un autre problème que se propose de résoudre l'invention, est de simplifier l'articulation des ailettes sur les parties correspondantes de l'anneau. A cet égard, on rappelle que les moyens d'articulation des ailettes sont constitués par des parties hémisphériques mâles formées à chaque extrémité, en débordement du profil de chaque ailette et aptes à coopérer avec des cavités hémisphériques complémentaires.

Selon l'invention et compte-tenu du problème posé à résoudre, les cavités hémisphériques sont formées dans des plots rapportés intégrés dans l'épaisseur de l'anneau et faisant office de palier.

Avantageusement, les plots sont cylindriques et fixés dans des logements complémentaires formés dans l'épaisseur de l'anneau.

Un autre problème que se propose de résoudre l'invention, est d'optimiser le couple d'articulation.

Un tel problème est résolu en ce que les plots sont en céramique mono cristalline ou tout autre matériau ayant un faible coefficient de frottement.

A noter que cette solution de paliers rapportés pour l'articulation des ailettes, s'avère particulièrement intéressante pour rationnaliser la fabrication et pour obtenir des états de surface de qualité excellente. En outre, il est possible de choisir le matériau le plus approprié qui notamment, peut être différent du matériau constituant l'anneau et/ou l'ailette.

L'invention est exposée, ci-après plus en détail à l'aide des dessins annexés, dans lesquels :
La figure 1 est une vue en perspective de la valve cardiaque, selon les caractéristiques de l'invention.
La figure 2 est à grande échelle, une vue partielle en coupe, montrant le profil de l'empreinte formée à la périphérie interne de l'anneau, et destinée à coopérer avec le profil des ailettes en position de fermeture.
La figure 3 est une vue correspondante à la figure 2 en position d'ouverture des ailettes.

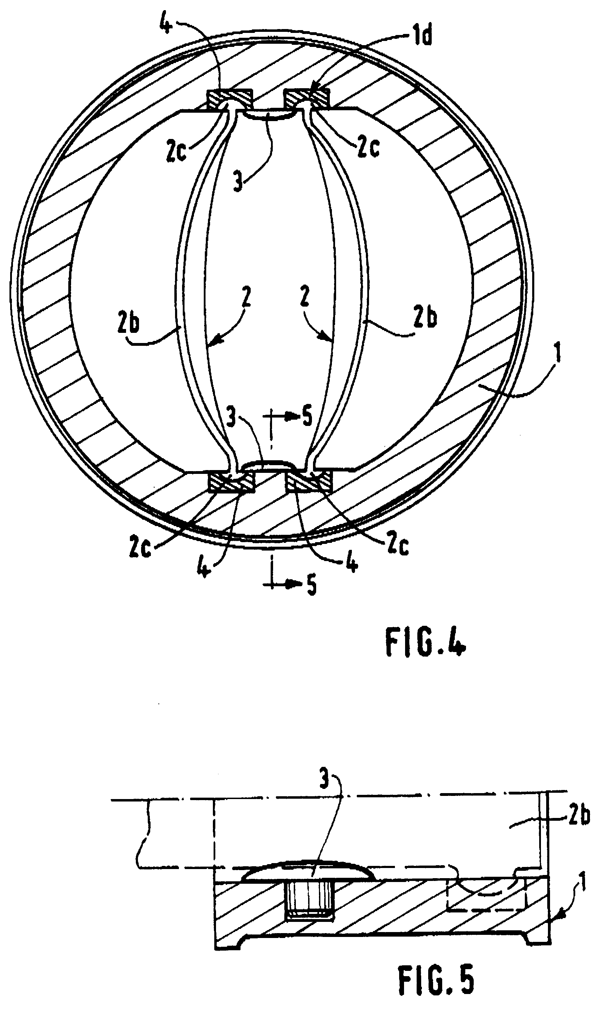

La figure 4 est à grande échelle, une vue en coupe transversale considérée selon la ligne 4.4 de la figure 1.
La figure 5 est à grande échelle, une vue en coupe transversale considérée selon la ligne 5.5 de la figure 4.

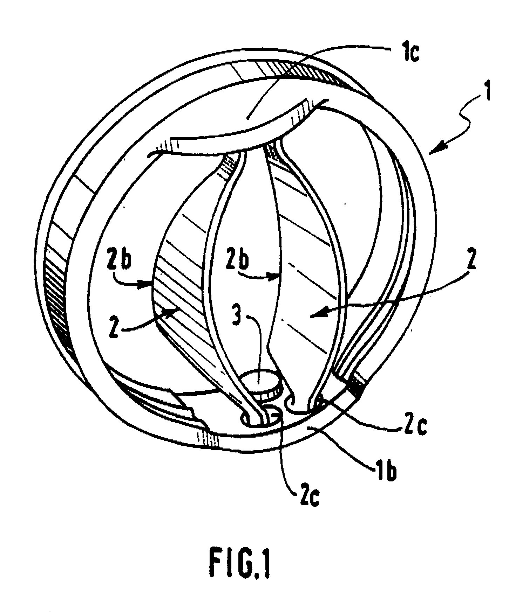

Comme le montre la figure 1, la valve artificielle comprend un anneau (1) à l'intérieur duquel sont articulés deux ailettes identiques (2). L'anneau (1) présente des agencements internes (1b) (1c) aptes à assurer l'articulation des ailettes (2), selon une position d'ouverture maximale, et délimiter, en combinaison avec le profil des ailettes, trois aires équivalentes. Ces agencements internes (1b) (1c) permettent, en position de fermeture des ailettes (2), leur intégration à l'intérieur de l'anneau.

Dans ce but, et comme il ressort de l'enseignement du brevet FR 2663533, ces agencements (1b) (1c) sont constitués par deux parties diamétralement opposées, formées directement en débordement de l'une des faces de l'anneau, du côté de la face d'admission de la valve. Ces parties (1b) (1c) assurent le déport du mécanisme d'articulation des ailettes (2), qui en position ouverte, ne dépassent le plan de l'anneau que d'une valeur réduite.

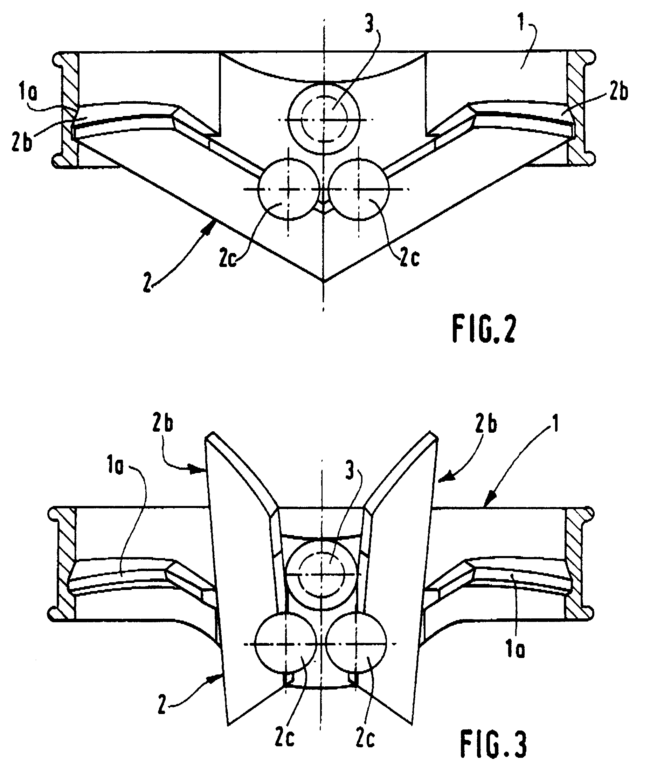

Suivant une caractéristique à la base de l'invention l'anneau (1) présente un agencement périphérique interne (1a) apte à assurer une partie ou surface d'appui linéaire au bord profilé externe des ailettes (2) en position de fermeture. Cet agencement est déterminé pour créer un phénomène d'amortissement en ayant pour objectif de réduire les bruits et tout phénomène d'usure. Dans ce but, et comme le montre la figure 2, cet agencement (1a) est constitué par une empreinte en creux, correspondant au profil externe du bord (2b) de l'ailette (2). Cette empreinte (1a) est formée de part et d'autre de la zone d'articulation des ailettes (2). La section transversale de l'empreinte (1a) est engendrée par le profil extérieur des ailettes lors de leur pivotement et déplacement en position de fermeture en créant un effet de butée.

Suivant une autre caractéristique de l'invention, les moyens de blocage des ailettes, en position d'ouverture, sont constitués par une pastille rapportée (3). Cette pastille est fixée en débordement de la face interne de l'anneau, entre les parties d'articulation des ailettes (2). Notamment, les pastilles (3) sont rapportées au niveau des parties (1b) et (1c) de l'anneau (figure 3). Les pastilles sont réalisées dans le même matériau que celui constituant l'anneau (1), à savoir le titane ou alliage de titane recouvert d'une pellicule de carbone amorphe et isotrope. A noter que chacune des pastilles (3) peut être fixée sur l'anneau, par faisceaux d'électrons.

L'articulation de chacune des ailettes (2), au niveau des parties (1b) et (1c), s'effectue par des parties hémisphériques mâles (2c) formées à chaque extrémité et directement dans le prolongement des bords de chaque ailette. Chacune des parties hémisphériques (2c) est destinée à coopérer avec des cavités hémisphériques complémentaires, que présente l'anneau (1). Dans ce but, et selon une autre caractéristique de l'invention, les cavités hémisphériques (4a) sont formées dans des plots rapportés (4) intégrés dans l'épaisseur de l'anneau (1). Les plots (4) sont cylindriques et fixés dans des logements complémentaires (1d) formés dans l'épaisseur de l'anneau (1). Avantageusement, les plots (4) sont en matériau cristallin ou autre matériau à faible coefficient de frottement, ce qui permet d'optimiser le couple d'articulation.

Les avantages ressortent bien de la description.

## Revendications

1. Valve cardiaque artificielle du type de celle comprenant un anneau (1) à l'intérieur duquel sont articulées des ailettes incurvées (2), l'anneau présentant des agencements diamétralement opposés (1b-1c) aptes à assurer le déport des moyens d'articulation des ailettes, pour qu'en position d'ouverture maximum d'environ au moins 85°, ces dernières ne débordent que d'une valeur réduite dans le plan de l'anneau en délimitant, en combinaison avec le profil des ailettes, trois aires équivalentes permettant un flux central axial et laminaire, les moyens d'articulation desdites ailettes étant intégrés dans l'épaisseur de l'anneau, de sorte que les extrémités des ailettes, au niveau des agencements (1b-1c), sont écartées en délimitant un espace entièrement libre pour ne pas limiter et gêner le flux central, caractérisée en ce que:
l'anneau (1) présente un agencement périphérique interne sous forme d'une empreinte en creux (1a), correspondant au profil externe du bord de l'ailette (2) et dont la section transversale est constituée par un profil courbe engendré par le déplacement du bord externe des ailettes en position de fermeture et en créant un effet de butée.

2. Valve selon la revendication 1, caractérisée en ce que les agencements (1b) (1c) présentent des moyens de blocage en position d'ouverture des ailettes (2), sous forme d'une pastille (3) rapportée fixée en débordement de la face interne de l'anneau (1), entre les parties d'articulation desdites ailettes (2).

3. Valve selon la revendication 1, dont les moyens d'articulation des ailettes (2) sont constitués par des parties hémisphériques mâles (2c) formées à chaque extrémité, en débordement du profil de chaque ailette et aptes à coopérer avec des cavités hémisphériques complémentaires, caractérisée en ce que les cavités hémisphériques (4a) sont formées dans des plots rapportés (4) intégrés dans l'épaisseur de l'anneau (1).

4. Valve selon la revendication 3, caractérisée en ce que les plots (4) sont cylindriques et fixés dans des logements complémentaires (1d) formés dans l'épaisseur de l'anneau (1).

5. Valve selon la revendication 3, caractérisée en ce que les plots (4) sont dans un matériau ayant un faible coefficient de frottement, notamment en céramique mono cristalline.

## Claims

1. Artificial heart valve of the type comprising a ring (1) which encloses hinged curved vanes (2), the ring having diametrically opposite features (1b-1c) designed to offset the means of hinging the vanes so that, in their maximum opened position of at least 85ø, the latter only project beyond the plane of the ring by a small amount thus delimiting, in conjunction with the shape of the vanes, three equivalent areas allowing a central axial laminar flow, the means of hinging the said vanes being integrated in the thickness of the ring so that the ends of the vanes at the level of features (1b-1c) are spread apart, thus delimiting a space which is completely unobstructed in order not to limit or interfere with the central flow characterised in that: The ring (1) has an internal peripheral feature in the form of a hollow imprint (1a) which matches the external shape of the edge of the vane (2) and of which the cross-section consists of a curved profile produced by moving the external edge of the vanes to their closed position, thus creating an end-stop effect.

2. Valve as claimed in claim 1, characterised in that the features (1b) (1c) have means of locking the vanes (2) in their opened position in the form of a separately mounted pad (3) fixed so that it projects beyond the internal surface of the ring (1) between the hinged parts of the said vanes (2).

3. Valve as claimed in claim 1 of which the means of hinging the vanes (2) consist of parts (2c) having a male hemispherical shape at each end which project beyond the profile of each vane and are suitable to cooperate with matching hemispherical cavities, characterised in that the hemispherical cavities (4a) are formed in separately mounted studs (4) integrated in the thickness of the ring (1).

4. Valve as claimed in claim 3, characterised in that the studs (4) are cylindrical and fixed in matching receptacles (1d) formed in the thickness of the ring (1).

5. Valve as claimed in claim 3, characterised in that the studs (4) are made of a material having a low coefficient of friction, particularly a monocrystalline ceramic.

## Patentansprüche

1. Künstliche Herzklappe nach Art derjeniger mit einem Ring (1), innerhalb dessen gebogene Flügel (2) gelenkig gelagert sind, wobei der Ring diametral entgegengesetzte Vorkehrungen (1b-1c) aufweist, die geeignet sind, den Versatz der Gelenkvorrichtungen der Flügel zu gewährleisten, damit letztere bei maximal geöffneter Stellung von ca. mindestens 85° in der Ringebene nur mäßig vorstehen und dabei in Verbindung mit dem Profil der Flügel drei gleiche Zonen begrenzen, die einen axialen und laminaren zentralen Fluß ermöglichen, wobei die Gelenkvorrichtungen der Flügel in den Ring integriert sind, dergestalt daß die Endstücke der Flügel auf Höhe der Vorkehrungen (1b-1c) auseinandergespreizt werden und dabei einen gänzlich freien Raum begrenzen, um den mittleren Fluß nicht einzuschränken bzw. zu behindern, dadurch gekennzeichnet, daß:
der Ring (1) am inneren Rand eine Vorkehrung in Form einer Vertiefung (1a) aufweist, die dem Außenprofil des Flügelrands (2) entspricht und deren Querschnitt durch ein Bogenprofil gebildet wird, das durch die Verschiebung des Außenrandes der Flügel in Schließstellung entsteht und dabei einen Anschlageffekt erzeugt.

2. Klappe nach Anspruch 1, dadurch gekennzeichnet, daß die Vorkehrungen (1b) (1c) in Offenstellung der Flügel (2) Blockiervorrichtungen in Form eines angesetzten Plättchens (3) aufweisen, das über die Innenfläche des Rings (1) hinausragend zwischen den gelenkigen Teilen der Flügel (2) befestigt ist.

3. Klappe nach Anspruch 1, bei der die Gelenkvorrichtungen der Flügel (2) aus halbkugelförmigen männlichen Teilen (2c) bestehen, die an jedem Ende über das Profil des jeweiligen Flügels hinausragen und geeignet sind, mit komplementären halbkugelförmigen Aushöhlungen zusammenzuwirken, dadurch gekennzeichnet, daß die halbkugelförmigen Aushöhlungen (4a) in angesetzten Klötzchen (4) ausgeformt sind, welche in den Ring (1) integriert sind.

4. Klappe nach Anspruch 3, dadurch gekennzeichnet, daß die Klötzchen (4) zylindrisch und in im Ring (1) ausgeformten ergänzenden Sitzen (1d) befestigt sind.

5. Klappe nach Anspruch 3, dadurch gekennzeichnet, daß die Klötzchen (4) aus einem Material bestehen, das einen geringen Reibbeiwert besitzt, insbesondere aus monokristalliner Keramik.
